# EUROPEAN PATENT APPLICATION

(11) **EP 3 263 144 A1**
(43) Date of publication of application: **03.01.2018**
(21) Application number: 16001466.8
(22) Date of filing: 30.06.2016
(51) Int. Cl.: A61L 27/24, A61L 27/36, A61L 27/38, A61L 27/54

(54) **A METHOD FOR PREPARING A MATERIAL FOR SOFT TISSUE REGENERATION AND A MATERIAL FOR SOFT TISSUE REGENERATION**

(71) Applicant: Zurek, Jacek, 42-612 Tarnowskie Gory (PL); Gedrange, Tomasz, 50-422 Wroclaw (PL); Dominiak, Marzena, 55-041 Tyniec Maly (PL)
(72) Inventor: Zurek, Jacek, 42-612 Tarnowskie Gory (PL); Gedrange, Tomasz, 50-422 Wroclaw (PL); Dominiak, Marzena, 55-041 Tyniec Maly (PL)
(74) Representative: Gizinska-Schohe, Malgorzata

(57) **Abstract**

A material for soft tissue regeneration, preferably for procedures in an oral cavity, characterized in that it comprises a cross-linked thin collagen membrane, obtained from the food industry, with the thickness between 1 and 2 mm, preferably enriched with deposited cells, medicinal products and/or medicinal articles.

## Description

The subject matter of the present invention is a method for preparing a material for soft tissue regeneration and a material for soft tissue regeneration, which is intended for application in various medical disciplines, such as dentistry, orthopaedics and cardiovascular surgery.

Polish patent specification No. PL140197 describes a method, which involves preparing a biostatic bovine fascia graft and requires harvesting fascia from young animals for slaughter, storing it in a mixture of ethyl alcohol and glutaraldehyde, rinsing it in a NaCl solution and then sterilising it. This method involves cutting the fascia flaps after rinsing in such a manner that they can form a mesh after being stretched.

Polish patent specification No. PL163877 describes a method for producing protein casings. In this method collagen obtained by known techniques is moulded into a thin-walled tube and then dried, hardened and plasticized. This process involves binding the collagen fibres with tanning substances and adding a gelling substance - carboxymethyl cellulose - to the tanning liquid at an increased temperature.

Moreover, Polish patent specification No. PL206261 describes the use of purified collagen taken from tissue containing natural collagen to produce a multi-layered sheet of membrane collagen for mucous membrane as well as a multi-layered collagen sheet of membrane for mucous membrane regeneration. The application of purified collagen material consists in the fact that membrane material contains a barrier layer which has a smooth outer surface and an opposite fibrous layer, as well as a matrix layer of collagen material formed through lyophilisation of the suspension of collagen material applied to the fibrous surface of the barrier layer, thanks to which the matrix layer adheres to the fibrous surface of the barrier layer and has an open cancellous-like structure. Such a multi-layered sheet of collagen membrane material functions as a barrier layer with a single smooth surface and an opposite fibrous surface and also as the matrix surface of the collagen material adhering to the fibrous surface of the barrier layer. The matrix layer has an open cancellous structure and contains collagen and/or a mix of collagen I and II.

Until now, dentists have used a procedure that involves harvesting autogenous connective tissue from the palate to augment gingiva, to widen keratinized gingiva zones and to cover gingival recessions. This procedure requires forming two surgical sites; the tissue harvesting site and the grafting site. The problem with harvesting connective tissue from the palate lies in the ambiguous anatomical-histological structure of hard palate mucosa (limited soft tissue thickness - the required thickness is 2.5 to 3 mm - high rate pegs, the presence of fatty and glandular tissue, the variable course of the palatine artery as well as various degrees of concavity of the palatal vault). The proper thickness is required so as to harvest a connective tissue graft of maximum uniformity with a recommended thickness of approximately 1 mm.

Using tissue from natural collagen offers an alternative to having to create two surgical sites. Moreover, it has a positive influence on wound healing and tissue integration. This type of tissue is used in orthopaedics, ophthalmology and other fields of medicine.

One inconvenience associated with these techniques is the need to use natural collagen tissue in lyophilised form, which makes application difficult (they require hydration beforehand) and prolongs healing time.

The purpose and task of the invention is to provide a method for obtaining material for soft tissue regeneration that can be applied directly to a treated surface (defect) in such fields of medicine as dentistry, orthopaedics and vascular surgery.

This goal has been achieved by obtaining cross-linked, i.e. hydrated, thin collagen membrane of zoonotic origin, which is isolated from the skin (hides) or tendons of livestock, such as cows or pigs.

The subject matter of the present invention is a method for preparing a material for soft tissue regeneration, characterized in that fibrous connective tissue of zoonotic origin is chemically treated for hydrolysing non-collagen proteins, and mechanically treated for the purpose for defibrating thereof into a homogeneous plastic mass. After filtering the plastic mass is formed into a thin collagen membrane with the thickness of between 1 and 2 mm, that is then dried and hardened. The collagen membrane is then cut into strips, immersed in a physiological saline solution and sterilised, thereby producing a cross-linked thin collagen membrane of zoonotic origin as a final product.

Preferably the fibrous connective tissue is taken from the hides or tendons of livestock, such as cows or pigs.

The hydrolysation of non-collagen proteins preferably is conducted with acid, preferably hydrochloric acid, which is then neutralised with a base, preferably sodium hydroxide.

Preferably the formed collagen membrane is dried at a temperature of between +20 and +60°C, preferably at a temperature of +40°C.

The collagen membrane is preferably cut into strips between 3 and 250 mm in width and between 20 and 500 mm in length, more preferably into strips of 8 mm x 40 mm in size.

The physiological saline solution preferably comprises 0.9% by weight of NaCl solution.

The cross-linked thin collagen membrane preferably is placed in a translucent glass bottle or a plastic container.

The cross-linked thin collagen membrane preferably is sterilised with a radiation dose of 10-50 kGy, more preferably 25 kGy, or the cross-linked thin collagen membrane preferably is sterilised with hydrogen peroxide.

The subject matter of the present invention also includes a material for soft tissue regeneration characterized in that the material comprises a cross-linked thin collagen membrane of zoonotic origin obtained by the method as described above.

Preferably the membrane on its surface has growth factors, preferably platelet-rich plasma (PRP).

Preferably the membrane on its surface has added cells, preferably fibroblasts.

Preferably the surface membrane has an additional medicinal product, preferably an antibiotic such as doxycycline, or an antiseptic such as chlorhexidine.

One advantage of the soft tissue regeneration material obtained by the method of the present invention is that after it has been taken out of its packaging it can be applied immediately to the treated surface or defect in such medical disciplines as dentistry, orthopaedics and vascular surgery. The cross-linked thin collagen membrane of zoonotic origin obtained by the method of the present invention maintains its original properties, i.e. the shape and elasticity of the native tissue, and does not require taking any additional steps before applying thereof. Another advantage of such a solution is the shorter time needed for grafting the membrane as well as faster integration thereof with the body of the recipient and faster healing of the trauma or defect.

### Example I

Fibrous connective tissue of zoonotic origin was obtained from the food industry. Bovine hides were chemically treated with the aim of hydrolysing non-collagen proteins in a 36% by weight of HCI solution, with a pH of 1-2 for 48 hours. They were then neutralised with a 0.1N NaOH solution and the salts obtained as a result were washed out with water many times. The bovine hides were then mechanically treated. This involved grinding the fibrous connective tissue into fragments 1 to 5 mm in size with the purpose of defibrating them so as to obtain a homogeneous plastic mass containing a collagen solution, that after filtration is maintained at a temperature of between +2°C and +8°C for a minimum of 12 hours, preferably for approximately 24 hours. This material was then poured onto metal moulds to form a thin-walled membrane with the thickness of between 1 and 2 mm, that was then dried and hardened at a temperature of +40°C for a period of 24 hours. The collagen membrane produced in this way was cut into strips 8 mm x 40 mm in size, placed in a translucent glass 5 ml bottle, containing 3 ml of sterile physiological saline solution, tightly sealed and then placed in air-tight outer packaging and sterilised with a 25 kGy dose of radiation. After it had been sterilised and delivered to the recipient, the material was removed from the packaging in the operating theatre using clean surgical techniques. Then, after cutting it to the appropriate size and preparing the donor site with the tunnel technique it was grafted onto the site of the gingival defect at the root surface so that that the material could be covered with a coronally advanced muco-gingival flap.

### Example II

Likewise in Example I the soft tissue regeneration material was obtained the chemical treatment of bovine hides to hydrolyse non-collagenous proteins, and then the mechanical treatment thereof to defibration so as to produce a homogenous plastic mass, which after filtering was stored at a temperature of +2°C do +8°C for a minimum of 12 hours. A thin-walled membrane between 1 and 2 mm thick was thereby formed, which was then dried and hardened. The membrane obtained in this way was inserted inside an inner packaging containing 3 ml of physiological salt, sealed and placed in an outer packaging, which was then sealed air tight with a welding tool. The entire material was then sterilised with a radiation dose of 25 kGy. After sterilisation the membrane was stored at room temperature.

Then, the cells of the connective tissue proper - fibroblasts - were applied to the sterilised collagen membrane with a pipette. The material with the cells was placed in a culture bottle in a culture medium and stored in a CO₂ incubator for a period of 12 days. The culture medium - physiological saline - i.e. a 0.9% by weight of NaCI solution - was changed every 48 hours. Following culturing, the material with deposited cells was placed in a sterile 10 ml container in a culture medium and stored at a temperature of between +4°C and +8°C for up to 48 hours until the moment it was used.

## Claims

1. A method for preparing a material for soft tissue regeneration, **characterized in that** fibrous connective tissue of zoonotic origin is chemically treated for hydrolysing non-collagen proteins, and mechanically treated for defibrating thereof into a homogeneous plastic mass, a thin collagen membrane with the thickness between 1 and 2 mm is formed of the homogenous plastic mass after filtering thereof, the membrane is dried and hardened, and next the collagen membrane is cut into strips, immersed in a physiological saline solution and sterilised to obtain a cross-linked thin collagen membrane of zoonotic origin as a final product.

2. The method according to claim 1 wherein the fibrous connective tissue are hides or tendons of livestock, such as cows or pigs.

3. The method according to claim 1 wherein the hydrolysing non-collagen protein is conducted with acid, preferably hydrochloric acid, which is then neutralised with a base, preferably sodium hydroxide.

4. The method according to claim 1 wherein the formed collagen membrane is dried at a temperature of between +20°C and +60°C, preferably at a temperature of +40°C.

5. The method according to claim 1 wherein the collagen membrane is cut into strips between 3 and 250 mm in width and between 20 and 500 mm in length, preferably into strips of 8 mm x 40 mm in size.

6. The method according to claim 1 wherein the physiological saline solution comprises 0.9% by weight of NaCl solution.

7. The method according to claim 1 wherein the cross-linked thin collagen membrane is placed in a translucent glass bottle or a plastic container.

8. The method according to claim 1 wherein the cross-linked thin collagen membrane is sterilised with a radiation dose of 10 to 50 kGy, preferably 25 kGy.

9. The method according to claim 1 wherein the cross-linked thin collagen membrane is sterilised with hydrogen peroxide.

10. A material for soft tissue regeneration **characterized in that** it comprises a cross-linked thin collagen membrane of zoonotic origin prepared by the method according to claim 1.

11. The material according to claim 10 wherein the membrane on its surface has growth factors, preferably platelet-rich plasma (PRP).

12. The material according to claim 10 wherein the membrane on its surface has added cells, preferably fibroblasts.

13. The material according to claim 10 wherein the membrane on its surface has an added medicinal product, preferably an antibiotic such as doxycycline, or preferably an antiseptic such as chlorhexidine.
